(19) Europäisches Patentamt

European Patent Office

Office européen des brevets

(11) **EP 1 632 220 A1**

(12) **DEMANDE DE BREVET EUROPEEN**

(43) Date de publication:
08.03.2006 Bulletin 2006/10

(51) Int Cl.:
*A61K 8/87* (2006.01)    *A61K 8/36* (2006.01)
*A61Q 5/04* (2006.01)

(21) Numéro de dépôt: 05291361.3

(22) Date de dépôt: 24.06.2005

(84) Etats contractants désignés:
AT BE BG CH CY CZ DE DK EE ES FI FR GB GR
HU IE IS IT LI LT LU MC NL PL PT RO SE SI SK TR
Etats d'extension désignés:
AL BA HR LV MK YU

(30) Priorité: 01.07.2004 FR 0451399

(71) Demandeur: L'OREAL
75008 Paris (FR)

(72) Inventeurs:
• Rollat-Corvol, Isabelle
75017 Paris (FR)
• Gawtrey, Jonathan
92100 Boulogne (FR)

(74) Mandataire: Bourdeau, Françoise
L'OREAL - D.I.P.I.
25-29 Quai Aulagnier
92600 Asnières (FR)

(54) **Procédé de déformation permanente des cheveux mettant en oeuvre des polymères filmogènes élastomères**

(57) La présente invention concerne un procédé déformation permanente des fibres kératiniques, en particulier des cheveux, ledit procédé étant notamment utilisable dans les salons de coiffure, professionnels ou par des particuliers, via la commercialisation de kits.

EP 1 632 220 A1

**Description**

La présente invention concerne un procédé déformation permanente des fibres kératiniques, en particulier des cheveux, ledit procédé étant notamment utilisable dans les salons de coiffure, professionnels ou par des particuliers, via la commercialisation de kits.

Au sens de la présente invention, on entend par «procédé de déformation permanente », tout procédé durable dans le temps, de mise en forme des cheveux, de frisage, de défrisage ou de décrêpage.

On entend par « fibres kératiniques », en particulier, les cheveux, les cils, les sourcils, et surtout les cheveux.

On sait que la technique la plus usuelle pour obtenir une déformation permanente des cheveux consiste, dans un premier temps, à réaliser l'ouverture des liaisons disulfures - S-S- de la kératine (cystine) à l'aide d'une composition réductrice, contenant un agent réducteur (étape de réduction) puis, après avoir de préférence, rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème des techniques de permanentes connues à ce jour est que leur application répétée sur les cheveux induit à la longue une altération progressive de la qualité du cheveu, et notamment une altération progressive et marquée de la brillance et des propriétés cosmétiques du cheveu, en particulier au niveau de la douceur des fibres qui ont tendance à devenir de plus en plus rêches ainsi qu'au niveau de leur démêlage, les cheveux devenant de plus en plus difficiles à démêler. Cette altération est en outre particulièrement accentuée lorsque l'étape de fixation de l'opération de déformation permanente est effectuée à l'aide d'un bromate.

Pour limiter cette altération des cheveux, il a déjà été proposé d'introduire directement, dans la composition réductrice, des agents de conditionnement. Par exemple, les demandes de brevet japonais H2-250814 et H9-151120 décrivent des compositions réductrices contenant des silicones aminées, pouvant éventuellement se présenter sous la forme de microémulsion.

Cependant, les procédés de déformation permanente des cheveux utilisant de telles compositions ne donnent pas encore entière satisfaction, dans la mesure où le degré, la qualité et la nervosité de frisure sont généralement insuffisants et éphémères, comme si l'agent de conditionnement, directement associé à l'agent réducteur, freinaient l'activité de ce dernier.

Le problème posé par l'invention est de fournir un procédé de déformation permanente des fibres kératiniques, notamment des cheveux, qui réduise la dégradation mécanique et/ou cosmétique des cheveux, tout en apportant un degré, une qualité et une nervosité de frisure satisfaisants.

La Demanderesse a découvert, de manière surprenante et inattendue, que l'utilisation de polymères filmogènes élastomères, pour la protection des fibres kératiniques, lors de traitements de mise en forme permanente ou d'opération de défrisage conduisait à une amélioration des propriétés mécaniques et cosmétiques des cheveux.

L'invention se rapporte encore à un procédé de traitement cosmétique de mise en forme permanente ou de défrisage des fibres kératiniques tel qu'il consiste à appliquer sur les fibres kératiniques une composition contenant un ou plusieurs polymères filmogènes élastomères.

De préférence, le polymère filmogène élastomère entrera dans la composition réductrice utilisée lors de la mise en forme de permanentes.

Il est connu que les cheveux régulièrement soumis à des opérations de permanentes perdent leurs propriétés mécaniques. Ces cheveux permanentés sont plus cassants, ils sont difficiles à mettre en forme, de plus il est difficile d'obtenir une coloration uniforme sur des cheveux permanentés.

Il a également été remarqué que les traitements cosmétiques visant à défriser les matières kératiniques et en particulier les cheveux, ont également pour conséquence d'altérer les propriétés mécaniques desdites matières kératiniques.

La demanderesse a ainsi montré que l'utilisation de compositions contenant des polymères filmogènes élastomères lors de la mise en forme de permanentes ou d'opération de défrisages des cheveux permet de limiter voire d'annuler les effets dégradants des permanentes.

La présente demande a pour objet une composition cosmétique comprenant une association, dans un milieu cosmétiquement acceptable, d'au moins un polymère filmogène élastomérique, choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75%, après un allongement de 150 %,
(c) une recouvrance ($R_{300}$) à 300 secondes supérieure à 80%.

et d'un composé choisi parmi les composés réducteurs de la kératine et les composés fixateurs de la kératine.

La présente demande concerne encore l'utilisation de cette composition cosmétique pour mettre en oeuvre une mise en forme permanente ou un défrisage, il pourra s'agir d'une composition réductrice ou d'une composition fixatrice, de préférence oxydante.

La présente demande concerne encore un dispositif à plusieurs compartiments, ou "kit" tel que l'un des compartiments comprend une composition réductrice contenant une association, dans un milieu cosmétiquement acceptable, d'au moins un polymère filmogène élastomérique, choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée (R;) au moins égale à 75%, après un allongement de 150%,
(c) une recouvrance ($R_{300}$) à 300 secondes supérieure à 80%.

et d'un composé réducteur de la kératine et un dispositif à plusieurs compartiments, ou "kit" tel que l'un des compartiments comprend une composition fixatrice contenant une association, dans un milieu cosmétiquement acceptable, d'au moins un polymère filmogène élastomérique, choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75%, après un allongement de 150%,
(c) une recouvrance ($R_{300}$) à 300 secondes supérieure à 80%.

et d'un composé fixateur de la kératine.

Un autre objet de la présente invention comprend un procédé de traitement cosmétique de mise en forme permanente ou de défrisage des fibres kératiniques, en particulier des cheveux, tels qu'il consiste à appliquer sur ces fibres, une composition cosmétique comprenant l'association, dans un milieu cosmétiquement acceptable, d'au moins un polymère filmogène élastomérique choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75 %, après un allongement de 150%,
(c) une recouvrance ($R_{300}$) à 300 secondes supérieure à 80 %.

et d'un composé choisi parmi les composés réducteurs de la kératine et les composés fixateurs de la kératine.

Ledit polymère filmogène élastomère peut être utilisé en association avec tous les composés réducteurs de la kératine ou avec tous les composés fixateurs de la kératine classiques dans toutes compositions cosmétiques classiques pour mettre en oeuvre une permanente ou un défrisage.

De préférence, les polymères filmogènes élastomères seront utilisés pour ou dans des compositions réductrices pour la mise en oeuvre de permanentes ou pour la mise en oeuvre d'un défrisage.

DESCRIPTION DETAILLEE DES MODES DE REALISATION DE L'INVENTION

Au sens de la présente invention, on entend par film obtenu par séchage à température ambiante (22°C $\pm$ 2°C) et à un taux d'humidité relative de 55 % $\pm$ 5%, le film obtenu dans ces conditions à partir d'un mélange à 6 % de matière active (m.a.) de polymère filmogène élastomérique dans un mélange 30 % en poids d'éthanol et de 70 % en poids d'eau, par rapport au poids total alcool+eau, la quantité de mélange étant adaptée pour obtenir dans une matrice en téflon, un film d'épaisseur de 500 $\mu$m $\pm$ 50 $\mu$m. Le séchage est poursuivi jusqu'à ce que le poids du film n'évolue plus, ce qui représente environ 12 jours. Les polymères filmogène solubles ou partiellement solubles dans l'éthanol sont testés dans l'éthanol seul. Les autres polymères sont testés dans l'eau seule, sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22°C $\pm$ 2 °C et un taux d'humidité relative de 50 % $\pm$ 5 %.

Les éprouvettes sont étirées à la vitesse de 20mm/min et la distance entre les mors est de 50 $\pm$ 1 mm.

Pour déterminer la recouvrance instantanée (R;), on procède comme suit :

- on étire l'éprouvette de 150 % ($e_{max}$) c'est-à-dire 1,5 fois sa longueur initiale ($l_0$),
- on relâche la contrainte en imposant une vitesse de retour égale à la vitesse de traction, soit 20mm/min, et on mesure l'allongement de l'éprouvette en pourcentage, après retour à charge nulle ($e_i$).

La recouvrance instantanée en % ($R_i$) est donnée par la formule ci-après :

$$R_i = ((e_{max} - e_i)/ e_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($e_{300s}$).

La recouvrance à 300 secondes en % ($R_{300s}$) est donnée par la formule ci-après :

$$R_{300s} = ((e_{max} - e_{300s})/ e_{max}) \times 100$$

De façon avantageuse, le ou les polymères de la composition selon l'invention, éventuellement associé(s) à un agent plastifiant et/ou un agent de filmification, sont tels qu'ils forment, dans les conditions des tests ci-dessus, un film d'allongement à la rupture allant de 800 % à 3000 % ; de recouvrance instantanée de 75 % à 100 % ; et une recouvrance à 300secondes allant de 85 % à 100 %.

Dans les compositions conformes à l'invention, le polymère filmogène élastomérique ou le mélange de polymères filmogènes élastomériques est, de préférence, présent à une concentration allant de 0,05 % à 20 % en poids, plus préférentiellement de 0,1 % à 15 % en poids, et par exemple de 0,25 % à 10 % en poids par rapport au poids total de la composition.

De manière avantageuse, le polymère filmogène élastomérique est choisi dans le groupe comprenant les polyuréthanes, les alcools polyvinyliques, les polymères comprenant au moins un motif (méth)acrylique, leurs associations. Il peut se présenter sous forme d'homopolymère ou de copolymère. En particulier, il se présente sous forme non réticulée dans la composition.

Si nécessaire, la composition peut, en outre comprendre un agent plastifiant et/ou un agent facilitant la filmification du ou des polymères élastomériques sur les matières kératiniques, dont la fonction est de modifier les propriétés du ou des polymères élastomériques. Un tel agent auxiliaire de filmification peut être choisi parmi tous les composés connus de l'homme du métier comme étant susceptibles de remplir la fonction recherchée, et être notamment choisi parmi les agents plastifiants et les agents de coalescence. Le ou les polymères filmogènes élastomériques, éventuellement associés à un agent plastifiant et/ou un agent facilitant la filmification sont aptes à former un film, après évaporation du milieu cosmétique. Cette évaporation peut être faite à l'air libre ou en apportant de la chaleur, par exemple à l'aide d'un séchoir.

Comme exemple d'agent plastifiant et/ou facilitant la filmification sur les matières kératiniques, on peut utiliser ceux décrits dans le document FR-A-2 782 917. En particulier, cet agent est choisi parmi les plastifiants ou agents de coalescence usuels, tels que:

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, le pentylène glycol,

- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- les esters d'acides notamment carboxyliques, tels que des citrates, des phtalates, des adipates, des carbonates, des tartrates, des phosphates, des sébaçates,
- leurs mélanges.

La quantité d'agent plastifiant et/ou d'agent de filmification peut être choisie par l'homme du métier sur base de ses connaissances générales, de manière à obtenir un système polymérique (polymères élastomériques + agent plastifiant et/ou agent de filmification) conduisant à un film ayant les propriétés mécaniques souhaitées, tout en conservant à la composition les propriétés cosmétiques recherchées. En pratique, cette quantité varie de 0,01 % à 25 % du poids total de la composition et mieux de 0,01 % à 15 %.

COMPOSITION REDUCTRICE

Les compositions réductrices pour la mise en oeuvre de permanentes peuvent consister en toute composition déjà connue en soi comme composition réductrice.

Plus particulièrement, les compositions réductrices utilisables pour la mise en oeuvre de permanentes contiennent, à titre d'agents réducteurs de la kératine, des sulfites et/ou des bisulfites, notamment d'alcalins, d'alcalino-terreux ou d'ammonium ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en $C_1$-$C_4$ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, et le thioglycérol. On peut également mentionner les réducteurs suivants : les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000 et les alkylaminomercaptoalkylamides décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Ces agents réducteurs de la kératine sont généralement mis en oeuvre dans des compositions cosmétiquement acceptables, lesquelles sont par ailleurs déjà bien connues en soi dans l'état de l'art existant des formulations frisantes destinées à réaliser la première étape (réduction) d'une opération de permanente. Ainsi, à titre d'additifs usuels et classiques, utilisables seuls ou en mélanges, on peut plus particulièrement mentionner les agents tensio-actifs de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensio-actifs non-ioniques du type hydroxypropyléther.

Lorsque la composition réductrice contient au moins un agent tensio-actif, celui-ci est généralement présent à une concentration maximale de 30% en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également, en plus du composé dérivé de polyguanidine, contenir un agent traitant de nature cationique, anionique, non-ionique ou amphotère.

Parmi les agents traitants particulièrement préférés, on peut notamment citer ceux décrits dans les demandes de brevets français n° 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonylalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy-(stéaroxydiméthicone), un copolymère polydiméthyl-siloxanedialkylammonium acétate ou un copolymère polydiméthylsiloxane polyalkylbétaïne décrits dans la demande de brevet britannique n° 2 197 352, des polysiloxanes organo modifiés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des polymères cationiques tels que ceux utilisés dans les compositions de brevets français n° 79 32078 (FR-A-2 472 382) et 80 26421 (FR-A-2 495 931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO$_2$/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylène-glycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C$_3$-C$_6$ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Dans des compositions réductrices de permanente, les agents réducteurs tels que ceux mentionnés ci-avant sont généralement présents à une concentration qui peut être comprise entre 1 et 30% en poids, et de préférence entre 5 et 20% en poids par rapport au poids total de la composition réductrice.

La composition réductrice peut se présenter sous la forme d'une lotion, épaissie ou non, d'une crème, d'un gel, ou de toute autre forme appropriée.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol ou de l'isopropanol, ou encore du glycérol à une concentration maximale de 20% par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes sous forme d'émulsions " lourdes ".'

Par exemple, pour obtenir une crème, on peut émulsionner une phase aqueuse contenant en solution le composé dérivé de polyguanidines et éventuellement d'autres ingrédients ou adjuvants, et une phase huileuse.

La phase huileuse peut être constituée par divers produits tels que l'huile de paraffine, l'huile de vaseline, l'huile d'amande douce, l'huile d'avocat, l'huile d'olive, des esters d'acides gras comme le monostéarate de glycéryle, les palmitates d'éthyl ou d'isopropyle, les myristates d'alkyle tels que les myristates de propyle, de butyle ou de cétyle. On peut en outre ajouter des alcools gras comme l'alcool cétylique ou des cires telles que par exemple la cire d'abeille.

On peut également utiliser des liquides ou des gels contenant des agents épaississants tels que des polymères ou des copolymères carboxyvinyliques qui " collent " les cheveux et les maintiennent dans la position lisse pendant le temps de pose.

Enfin, les compositions réductrices peuvent également contenir au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl)ω-hydroxyalkylamides décrits dans la demande de brevet européen EP 354 835, les disulfures des N-mono ou N,N-dialkylmercapto-4-butyramides décrits dans la demande de brevet EP 368 763, les disulfures des aminomercapto-alkylamides décrits dans la demande de brevet EP 432 000, et les disulfures des alkylaminomercaptoalkylamides décrits dans la demande de brevet EP 514 282. Ces disulfures sont généralement présents dans un rapport molaire de 0,5 à 2,5, et de préférence de 1 à 2, par rapport à l'agent réducteur (voir brevet US 3 768 490).

Les pH des compositions réductrices peuvent être ajustés classiquement par ajout, soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine (compositions réductrices carbonatées) ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

Les compositions comprenant, en association, au moins un polymère filmogène élastomère et au moins un composé réducteur choisi parmi les sulfites ou les bisulfites tels que définis ci-dessus, peuvent être appliquées de manière répétée sur les cheveux pour la mise en oeuvre de permanentes ou de défrisages sans que l'on observe une modification importante du comportement de ces cheveux, en particulier au niveau de leur aptitude à être par la suite correctement colorés.

En effet, on observe généralement que, sur les cheveux qui ont subi quelques opérations de permanente ou de défrisage (de l'ordre de trois au maximum), la coloration sera beaucoup plus prononcée que celle obtenue sur les mêmes cheveux, mais non permanentés. Ceci pose donc un problème dans tous les cas où l'opération de coloration est conduite sur une chevelure à l'origine permanentée mais qui, entre temps, a repoussé (mauvaise unisson entre les cheveux d'origine permanentés et les cheveux de repousse non permanentés).

On observe également que la coloration devient très difficile, voire impossible, si la chevelure à colorer a subi auparavant de nombreuses opérations de permanentes ou de défrisages, en particulier plus de cinq permanentes.

Il convient de remarquer que les compositions cosmétiques utilisées dans le cadre de l'invention sont aussi bien des compositions prêtes à l'emploi que des concentrés devant être dilués avant l'utilisation. Les compositions cosmétiques ne sont donc pas limitées à un domaine particulier de concentration des polymères filmogènes.

Généralement, dans les compositions cosmétiques utilisées, la concentration en polymères filmogènes élastomères est comprise entre 0,001 et 25% en poids, et de préférence, entre 0,1 et 10% en poids par rapport au poids total de la composition.

Ces compositions peuvent être conditionnées sous diverses formes, notamment dans des flacons éventuellement pourvus de pompe, des bouillottes, des tubes ou de simples pots pour une prise à la main. Les compositions conformes à l'invention peuvent se présenter sous la forme de crème, de gel, d'émulsion eau-dans-huile ou huile-dans-eau, de lotion, de spray, de mousse ou de cire.

Dans un mode de mise en oeuvre, le pH de la phase aqueuse est choisi dans la gamme allant de 2 à 11 et préférentiellement de 3 à 10 par exemple de 5 à 8.

Avantageusement, la composition présente une viscosité dynamique, mesurée à température ambiante et pression atmosphérique inférieure à 200 cps, et par exemple comprise entre 180 cps et 10 cps. Cette viscosité peut être mesurée par toute méthode connue et par exemple à l'aide d'un Rhéomat 180 , notamment à 25°C et sous un taux de cisaillement de 1s-1 .

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'invention peuvent être appliquées sur des matières kératiniques tels que les cheveux, peau, et cils à l'état sec ou humide.

A titre illustratif et non limatif, on donne ci-après des exemples concrets de réalisation de la composition selon l'invention.

Les quantités sont données en pourcentage massique et M.A. signifie matière active.

Exemple de composition permanente :

| | |
|---|---|
| Polyuréthane (NMDEA[1] / PTMO 2900[2]) / IPDI[3]) - 3 / 1 / 4 ) | 4% MA |
| Acide thioglycolique | 9g |
| Ammoniaque pH ajusté à 8.4 | pH 8.4 |
| Eau | qsp 100% MA |
| [1] - N-méthyldiéthanolamine [2] - Poly (tétraméthylène oxyde) ayant une masse moyenne en poids de 2900 [3] - Isophoronediisocanate | |

## Revendications

**1.** Composition cosmétique comprenant l'association, dans un milieu cosmétiquement acceptable, d'au moins un polymère filmogène élastomérique choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %

et d'un composé choisi parmi les composés réducteurs de la kératine et les composés fixateurs de la kératine.

**2.** Composition selon la revendication 1, **caractérisée en ce que** le polymère filmogène élastomérique est soluble dans un milieu aqueux ou hydroalcoolique.

**3.** Composition selon la revendication 1 ou 2, **caractérisée en ce qu'**entre 30 % et 80 % d'humidité relative, le taux d'allongement à la rupture du film obtenu soit compris entre 400 et 1200% et /ou sa recouvrance instantanée soit comprise entre 57 et 93%

**4.** Composition cosmétique selon la revendication 3 telle que la composition présente une viscosité dynamique, mesurée à température ambiante et pression atmosphérique inférieure à 200 cps.

**5.** Composition cosmétique selon l'une des revendications précédentes, telle que le polymère filmogène élastomère est choisi dans le groupe comprenant les polyuréthannes, les alcools polyvinyliques, les polymères comprenant au moins un motif (méth)acrylique, leurs associations.

**6.** Composition cosmétique selon la revendication 5, telle que le composé réducteur est choisi parmi les thiols et en particulier la cystéine et ses dérivés, la cystéamine et ses dérivés, l'acide thiolactique et ses esters, l'acide thioglycolique et ses esters.

**7.** Utilisation d'une composition cosmétique selon l'une des revendications 1 à 6 pour effectuer une mise en forme permanente ou un défrisage des fibres kératiniques.

**8.** Utilisation d'une composition cosmétique selon la revendication 7 telle que la composition cosmétique est une composition réductrice.

**9.** Utilisation d'une composition cosmétique selon l'une des revendications 1 à 6 telle que cette composition cosmétique est fixatrice pour effectuer une mise en forme permanente des fibres kératiniques.

**10.** Utilisation selon l'une des revendications 7 ou 8 telle que le polymère filmogène élastomérique choisi de telle sorte que le film obtenu par séchage de ce polymère, à température ambiante et à un taux d'humidité relative de 55 %, présente un profil mécanique défini par au moins :

    (a) un taux d'allongement à la rupture ($e_r$) supérieur ou égal à 800 %,
    (b) une recouvrance instantanée ($R_i$) au moins égale à 75 %, après un allongement de 150 %,
    (c) une recouvrance ($R_{300}$) à 300 secondes supérieure à 80 %,

représente de 0,001 à 25% en poids et de préférence de 0,1 à 10% en poids par rapport au poids total de la composition cosmétique.

**11.** Utilisation selon l'une des revendications 7 ou 8 telle que la composition cosmétique contient au moins un agent choisi parmi les tensioactifs non ioniques, anioniques, cationiques ou amphotères, les agents traitants non ioniques, anioniques, cationiques, ou amphotères.

**12.** Procédé de traitement de cosmétique de mise en forme permanente ou de défrisage des fibres kératiniques tel qu'il consiste à appliquer sur les fibres kératiniques une composition cosmétique comprenant une association dans un milieu cosmétiquement acceptable, d'au moins un polymère filmogène élastomérique choisi de telle sorte que le film obtenu par séchage de ce polymère, à température ambiante et à un taux d'humidité relative de 55 %, présente un profil mécanique défini par au moins :

    (a) un taux d'allongement à la rupture ($e_r$) supérieur ou égal à 800 %,
    (b) une recouvrance instantanée ($R_i$) au moins égale à 75 %, après un allongement de 150 %,
    (c) une recouvrance ($R_{300}$) à 300 secondes supérieure à 80 %

et d'un composé choisi parmi les composés réducteurs de la kératine et les composés fixateurs de la kératine.

**13.** Dispositif à plusieurs compartiments, ou "kit" **caractérisé en ce que** l'un des compartiments comprend une composition cosmétique réductrice contenant une association, dans un milieu cosmétiquement acceptable, d'au moins un polymère filmogène élastomérique choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %

et d'un composé réducteur de la kératine selon l'une des revendications 1 à 6.

**14.** Dispositif à plusieurs compartiments, ou "kit" **caractérisé en ce que** l'un des compartiments comprend une composition cosmétique fixatrice contenant une association, dans un milieu cosmétiquement acceptable, d'au moins un polymère filmogène élastomérique choisi de telle sorte que le film obtenu par séchage de ce polymère, à température ambiante et à un taux d'humidité relative de 55 %, présente un profil mécanique défini par au moins :

(a) un taux d'allongement à la rupture ($e_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %

et d'un composé fixateur de la kératine selon l'une des revendications 1 à 4.

**Office européen des brevets**

**RAPPORT DE RECHERCHE EUROPEENNE**

Numéro de la demande

EP 05 29 1361

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 815 350 A (OREAL) 19 avril 2002 (2002-04-19) * le document en entier * ----- | 1-14 | A61K7/09 |
| X | FR 2 833 960 A (OREAL) 27 juin 2003 (2003-06-27) * revendications 1-26; exemple 1 * ----- | 1-14 | |
| X | PATENT ABSTRACTS OF JAPAN vol. 001, no. 070 (C-019), 8 juillet 1977 (1977-07-08) & JP 52 034940 A (MATSUSHITA ELECTRIC WORKS LTD), 17 mars 1977 (1977-03-17) * abrégé * ----- | 1-14 | |
| A | US 6 410 004 B1 (HOESSEL PETER ET AL) 25 juin 2002 (2002-06-25) * le document en entier * ----- | 1-14 | |
| A | US 6 335 003 B1 (HOESSEL PETER ET AL) 1 janvier 2002 (2002-01-01) * le document en entier * ----- | 1-14 | DOMAINES TECHNIQUES RECHERCHES (Int.Cl.7) A61K |

Le présent rapport a été établi pour toutes les revendications

| Lieu de la recherche | Date d'achèvement de la recherche | Examinateur |
|---|---|---|
| Munich | 16 septembre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 29 1361

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-2005

| Document brevet cité au rapport de recherche | | Date de publication | Membre(s) de la famille de brevet(s) | | Date de publication |
|---|---|---|---|---|---|
| FR 2815350 | A | 19-04-2002 | AU | 9569201 A | 29-04-2002 |
| | | | EP | 1326908 A1 | 16-07-2003 |
| | | | WO | 0232978 A1 | 25-04-2002 |
| | | | JP | 2004511637 T | 15-04-2004 |
| | | | US | 2004052753 A1 | 18-03-2004 |
| FR 2833960 | A | 27-06-2003 | EP | 1323756 A1 | 02-07-2003 |
| | | | JP | 2003226733 A | 12-08-2003 |
| | | | US | 2004001798 A1 | 01-01-2004 |
| JP 52034940 | A | 17-03-1977 | AUCUN | | |
| US 6410004 | B1 | 25-06-2002 | CN | 1269374 A | 11-10-2000 |
| | | | EP | 1035144 A2 | 13-09-2000 |
| | | | JP | 2000336141 A | 05-12-2000 |
| US 6335003 | B1 | 01-01-2002 | CA | 2148805 A1 | 23-06-1994 |
| | | | DE | 4241118 A1 | 09-06-1994 |
| | | | WO | 9413724 A1 | 23-06-1994 |
| | | | EP | 0672076 A1 | 20-09-1995 |
| | | | ES | 2108415 T3 | 16-12-1997 |
| | | | JP | 8504454 T | 14-05-1996 |
| | | | JP | 3369180 B2 | 20-01-2003 |

EPO FORM P0460

Pour tout renseignement concernant cette annexe : voir Journal Officiel de l'Office européen des brevets, No.12/82